# EUROPEAN PATENT APPLICATION

(11) **EP 4 844 364 A1**
(43) Date of publication of application: **25.11.2026**
(21) Application number: 24933782.5
(22) Date of filing: 04.11.2024
(51) Int. Cl.: G01N 1/02

(54) **SAMPLE COLLECTION AND TESTING DEVICE, KIT AND TESTING METHOD**

(30) Priority: 01.04.2024 CN 202410388102
(71) Applicant: Shenzhen Yhlo Biotech Co., Ltd., Shenzhen, Guangdong 518116 (CN)
(72) Inventor: ZHANG, Sai, Shenzhen, Guangdong 518116 (CN); LI, Xiaodong, Shenzhen, Guangdong 518116 (CN); QIAN, Chungen, Shenzhen, Guangdong 518116 (CN); WANG, Gang, Shenzhen, Guangdong 518116 (CN)
(74) Representative: Ormrod, Beth Katie
(86) International application number: PCT/CN2024/129583
(87) International publication number: WO 2025/208857

(57) **Abstract**

The present application relates to the field of point-of-care testing, and particularly relates to a sample collection and testing device, a kit, and a testing method. The sample collection and testing device includes a collection structure and a liquid storage structure. The collection structure has an accommodation chamber configured to accommodate a testing member. An end of the collection structure is configured to collect a sample. The collection structure is provided with a liquid inlet in communication with the accommodation chamber. The liquid storage structure has a liquid storage chamber and an access opening in communication with the liquid storage chamber. The liquid storage chamber is configured to store a diluent. The collection structure is configured to enter the liquid storage chamber through the access opening, be in interference fit with a chamber wall of the liquid storage chamber, and move along the chamber wall. After entering the liquid storage chamber, the collection structure can squeeze the diluent to cause the diluent to flush the sample to form a liquid mixture, and simultaneously squeeze the liquid mixture to enter the accommodation chamber through the liquid inlet and come into contact with the testing member. By providing the collection structure and the liquid storage structure, the above sample collection and testing device enables sample addition, sample mixing, and testing by the testing member to be integrated and performed in one step, thereby simplifying operation and improving testing efficiency.

## Description

### TECHNICAL FIELD

The present application relates to the field of point-of-care testing, and particularly relates to a sample collection and testing device, a kit, and a testing method.

### BACKGROUND

Fecal testing can be used for the auxiliary diagnosis of various diseases and can be used to determine whether a subject is infected with bacteria, viruses, or the like. Currently, fecal testing devices require additional steps, such as separately performing sample mixing and sample addition, resulting in complicated operation and reduced testing efficiency.

### SUMMARY

In view of this, there is a need to provide a sample collection and testing device, a kit, and a testing method to address the problems that existing fecal testing devices require additional steps such as sample mixing and sample addition, resulting in complicated operation and reduced testing efficiency.

In a first aspect, a sample collection and testing device is provided, including:
a collection structure having an accommodation chamber, wherein the accommodation chamber is configured to accommodate a testing member, an end of the collection structure is configured to collect a sample, and the collection structure is provided with a liquid inlet in communication with the accommodation chamber; and
a liquid storage structure having a liquid storage chamber and an access opening in communication with the liquid storage chamber, wherein the liquid storage chamber is configured to store a diluent, and the collection structure is configured to enter the liquid storage chamber through the access opening, be in interference fit with a chamber wall of the liquid storage chamber, and move along the chamber wall.

In an embodiment, the liquid storage structure includes a base and a pressurizing member. The base has the liquid storage chamber and the access opening. The pressurizing member is disposed in the liquid storage chamber and movably abuts against the chamber wall. The pressurizing member is provided therein with a through hole extending through the pressurizing member and in communication with the liquid storage chamber. The collection structure is configured to enter the liquid storage chamber and push the pressurizing member to move relative to the chamber wall.

In an embodiment, the base includes an upper base cover, a lower base cover, and a diluent tube;
the lower base cover includes a main body portion and an extension portion, the extension portion is connected to an outer periphery of the main body portion and extends in a direction away from the main body portion, the main body portion has a first placement chamber and a first opening in communication with the first placement chamber, the upper base cover has a second placement chamber and a second opening and a third opening that are in communication with the second placement chamber, the second opening and the third opening are oppositely disposed in an axial direction of the upper base cover, the diluent tube has the liquid storage chamber and the access opening, and the pressurizing member is disposed in the diluent tube and abuts against an inner wall of the diluent tube; and
the diluent tube extends through the first opening and is positioned in the first placement chamber, an outer wall of the diluent tube abuts against a chamber wall of the first placement chamber, the lower base cover extends through the third opening and is positioned in the second placement chamber, an outer periphery of the extension portion abuts against a chamber wall of the second placement chamber, and the access opening is at least partially exposed through the first opening and the second opening.

In an embodiment, the collection structure includes a collection body, a cover body, and an accommodation body, an end of the collection body is configured to collect the sample, the collection body is provided with the liquid inlet, and the accommodation body has a hollow portion extending therethrough in a length direction thereof; and
the collection body is detachably connected to one end of the hollow portion, and the cover body is detachably connected to the other end of the hollow portion, such that the collection body, the cover body, and the accommodation body jointly define the accommodation chamber.

In an embodiment, the collection structure further includes a filter body. The filter body abuts against the chamber wall of the accommodation body. The filter body is at least partially in contact with the liquid inlet and the testing member.

In an embodiment, the collection body is recessed inward in a direction towards a bottom end thereof to form a recess. The recess is in communication with both the accommodation chamber and the liquid inlet. The filter body is located in the recess, and the filter body abuts against a wall of the recess.

In an embodiment, a recess body of the recess includes a stepped portion and an abutting portion. The abutting portion is disposed at a top end of the stepped portion. The filter body abuts against both an upper surface of the stepped portion and an inner peripheral surface of the abutting portion. The filter body is located above the liquid inlet. The testing member abuts against an upper surface of the filter body.

In an embodiment, the cover body is provided with a retaining groove. After the cover body is connected to the accommodation body, the retaining groove is in communication with the accommodation chamber. The retaining groove is configured to fix the testing member.

In a second aspect, a kit is provided, including the sample collection and testing device as described in the first aspect.

In a second aspect, the testing method is provided, which uses the sample collection and testing device as described in the first aspect to test a sample. The testing method includes the following steps:
adhering a sample to the collection structure;
inserting the collection structure having the sample adhered thereto into the liquid storage chamber through the access opening, squeezing the diluent in the liquid storage chamber to cause the diluent to flush the sample to form a liquid mixture, and pressing the liquid mixture into the accommodation chamber to be in contact with the testing member; and
observing the testing member in the accommodation chamber of the collection structure and reading a testing result.

By providing the collection structure and the liquid storage structure, the above sample collection and testing device enables sample addition, sample mixing, and testing by the testing member to be integrated and performed in one step, thereby simplifying operation and improving testing efficiency. Specifically, after the collection structure collects a sample, the collection structure is caused to enter the liquid storage chamber through the access opening, and because the collection structure is in interference fit with the chamber wall of the liquid storage chamber, a sealed space is formed between the collection structure and the liquid storage chamber. During the process in which the collection structure instantaneously enters the liquid storage chamber, the pressure inside the liquid storage chamber rises sharply, forcing the diluent to form a rapid flow that flushes the sample on the exterior of the collection structure, such that the sample and the diluent are mixed to form a liquid mixture. When the collection structure continues to move relative to the liquid storage chamber, the internal space of the liquid storage chamber is reduced, thereby squeezing the liquid mixture to enter the accommodation chamber of the collection structure through the liquid inlet on the collection structure. After coming into contact with the liquid mixture, the testing member can automatically test the liquid mixture. Therefore, merely by inserting the collection structure into the liquid storage chamber of the liquid storage structure, sample addition, sample mixing, and testing can be achieved in one step, without requiring additional sample mixing and sample addition steps, thereby improving testing efficiency.

### BRIEF DESCRIPTION OF THE DRAWINGS

FIG. 1 is a perspective view of a sample collection and testing device provided in an embodiment of the present application.
FIG. 2 is a sectional view of a sample collection and testing device provided in an embodiment of the present application.
FIG. 3 is a partial sectional view of a sample collection and testing device provided in an embodiment of the present application.
FIG. 4 is an exploded schematic view of a collection structure in an embodiment of the present application.
FIG. 5 is a perspective view of a collection body of the collection structure in an embodiment of the present application.
FIG. 6 is an exploded schematic view of a liquid storage structure in an embodiment of the present application.

Description of reference number: 100, sample collection and testing device; 1, collection structure; 101, accommodation chamber; 102, testing member; 1021, sample pad; 1022, protein conjugate pad; 1023, nitrocellulose membrane; 1024, testing line; 1025, quality control line; 1026, water-absorbing pad; 103, liquid inlet; 11, collection body; 111, recess; 111a, stepped portion; 111b, abutting portion; 112, second engagement portion; 112a, second neck portion; 112b, second shoulder portion; 113, first groove; 12, cover body; 121, retaining groove; 122, first engagement portion; 122a, first neck portion; 122b, first shoulder portion; 13, accommodation body; 131, hollow portion; 132, first protrusion; 133, viewing window; 14, filter body; 15, toothed structure; 16, annular groove; 2, liquid storage structure; 21, liquid storage chamber; 22, access opening; 23, base; 231, upper base cover; 2311, second placement chamber; 2312, second opening; 2313, third opening; 232, lower base cover; 2321, main body portion; 2321a, first placement chamber; 2321b, first opening; 2322, extension portion; 2323, bent portion; 233, diluent tube; 2331, lapping portion; 24, pressurizing member; 241, through hole.

### DETAILED DESCRIPTION

To make the above objectives, features, and advantages of the present application more apparent and understandable, specific embodiments of the present application are described in detail below with reference to the accompanying drawings. Many specific details are set forth in the following description to facilitate a comprehensive understanding of the present application. However, the present application can be implemented in various ways different from those described herein, and those skilled in the art may make similar improvements without departing from the essence of the present application. Therefore, the present application is not limited to the specific embodiments disclosed below.

In the description of the present application, it should be understood that if such terms as "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial" , "radial", and "circumferential" appear, the orientations or positional relationships indicated by these terms are based on the orientations or positional relationships shown in the accompanying drawings, and are merely for facilitating the description of the present application and simplifying the description, rather than indicating or implying that the referred devices or elements must have the specific orientations or be constructed and operated in the specific orientations, and therefore cannot be construed as limiting the present application.

In addition, if such terms as "first" and "second" appear, these terms are used only for descriptive purposes and cannot be construed as indicating or implying relative importance or implicitly specifying the number of the indicated technical features. Accordingly, a feature defined by "first" or "second" may explicitly or implicitly include at least one such feature. In the description of the present application, if the term "a plurality of" appears, "a plurality of" means at least two, for example, two, three, etc., unless otherwise explicitly and specifically defined.

In the present application, unless otherwise explicitly specified and defined, if such terms as "mounted," "coupled," "connected," and "fixed" appear, these terms should be understood in a broad sense. For example, it may be a fixed connection, a detachable connection, or an integral connection; it may be a mechanical connection or an electrical connection; it may be a direct connection or an indirect connection through an intermediate medium; and it may be internal communication between two elements or an interaction relationship between two elements, unless otherwise explicitly defined. For those of ordinary skill in the art, the specific meanings of the above terms in the present application may be understood according to specific circumstances.

Fecal testing can be used for the auxiliary diagnosis of various diseases, and whether a subject is infected with bacteria, viruses, or the like can be determined by testing feces, thereby achieving the purpose of diagnosing diseases. Current fecal testing devices mainly include the following two types. In one type of fecal testing device, a sampling rod is first used to collect a certain amount of a fecal sample, and is then inserted into a collection tube containing a diluent. The fecal sample on the sampling rod is uniformly mixed with the diluent through steps such as scraping, shaking, and standing, and subsequently the sample liquid mixture is added dropwise into a sampling well on a test plate or a testing member. This method requires additional sample mixing and sample addition steps, involves complicated steps and a long time consumption, and is liable to cause liquid leakage during shaking of the liquid mixture and dropwise addition of the liquid mixture, thereby causing secondary contamination. In another type of fecal testing device, a structure such as a plug or a baffle is provided between the diluent and the testing member. A sampling rod is used to collect a certain amount of a fecal sample, and is then inserted into a structure containing the diluent. The structure is shaken to sufficiently mix the fecal sample with the diluent, and then an external force is further applied to cause the sampling rod to break the plug or baffle structure, so that the liquid mixture flows onto the testing member. Although this type of testing device does not require dropwise sample addition as does the preceding type of device, it still requires shaking for sample mixing before sample addition and requires an additional sample addition triggering action. Therefore, current fecal testing devices all require sample mixing and sample addition to be additionally and separately performed, and thus the testing steps are complicated, and the testing efficiency remains to be improved.

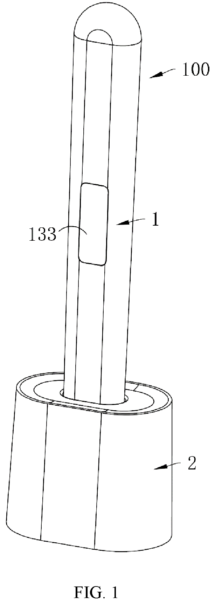

In view of the above problems, in a first aspect, referring to FIG. 1, an embodiment of the present application provides a sample collection and testing device 100, including a collection structure 1 and a liquid storage structure 2.

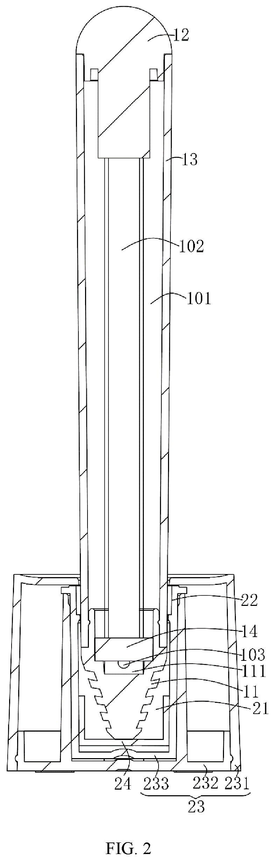

Referring to FIGS. 1 and 2, the collection structure 1 has an accommodation chamber 101. The accommodation chamber 101 is configured to accommodate a testing member 102. An end of the collection structure 1 is configured to collect a sample. The collection structure 1 is provided with a liquid inlet 103 in communication with the accommodation chamber 101. The liquid storage structure 2 has a liquid storage chamber 21 and a access opening 22 in communication with the liquid storage chamber 21. The liquid storage chamber 21 is configured to store a diluent. The collection structure 1 is configured to enter the liquid storage chamber 21 through the access opening 22, and be in interference fit with a chamber wall of the liquid storage chamber 21, and move along the chamber wall, so as to squeeze the diluent to cause the diluent to flush the sample to form a liquid mixture, and simultaneously squeeze the liquid mixture to enter the accommodation chamber 101 through the liquid inlet 103 and come into contact with the testing member 102. By providing the collection structure 1 and the liquid storage structure 2, the sample collection and testing device 100 of the present application does not require additional sample mixing and sample addition steps, and can automatically achieve sample mixing and sample addition, simplifying the testing steps and improving the testing efficiency.

Specifically, after collecting the sample, the collection structure 1 enters the liquid storage chamber 21 through the access opening 22. Since the collection structure 1 is in interference fit with the chamber wall of the liquid storage chamber 21, a sealed space is formed between the collection structure 1 and the liquid storage chamber 21. At the instant when the collection structure 1 enters the liquid storage chamber 21, the air pressure inside the sealed space is increased, and the pressure forces the diluent to form a rapid flow to flush the sample on the collection structure 1, thereby causing the sample and the diluent to be mixed to form the liquid mixture. As the collection structure 1 further moves relative to the chamber wall, the sealed space is reduced, and the liquid mixture is squeezed to flow through the liquid inlet 103 into the accommodation chamber 101 of the collection structure 1 and come into contact with the testing member 102. As such, by inserting the collection structure 1 into the liquid storage chamber 21, the effects of sample mixing, sample addition, and testing can be achieved simultaneously, so that additional steps such as shaking and sample addition are not required, thereby simplifying the testing steps to a great extent, and improving the testing efficiency.

Further, since the collection structure 1 is in interference fit with the liquid storage structure 2, after completion of the testing, the liquid mixture is completely stored in the sealed space formed by the collection structure 1 and the liquid storage structure 2, thereby avoiding secondary contamination caused by leakage of the liquid mixture and improving the safety and hygiene of the sample collection and testing device 100 of the present application.

Further, in the second type of fecal testing device, structures such as a baffle or a plug are used to isolate the diluent from the testing member, and when the baffle or the plug becomes loose or detached or is accidentally damaged, the diluent directly wets the testing member, causing the testing member to fail, thereby reducing the reliability of the testing device, and affecting the testing result. Whereas, in the sample collection device of the present application, the testing member 102 is disposed in the collection structure 1, the diluent is disposed in the liquid storage structure 2, and the testing member 102 and the diluent are separately provided, thereby avoiding the possibility of accidental contact between the testing member 102 and the diluent, reducing the possibility of failure of the testing member 102, and improving the reliability and stability of the testing device.

Further, in the two types of fecal testing devices described above, differences in operating habits among different subjects may cause inaccurate testing results; for example, different sample mixing manners may cause the fecal sample and the diluent to be mixed non-uniformly, resulting in poor dilution, and inaccurate control of a dropwise addition amount affects the testing precision. However, the sample collection and testing device 100 provided by the embodiments of the present application avoids the above problems, and when a subject inserts the collection structure 1 into the liquid storage chamber 21, the travel through which the collection structure 1 moves from entering the liquid storage chamber 21 to abutting against the bottom of the liquid storage chamber 21 is consistent, the compressed space is consistent, and the amount of the liquid mixture entering the accommodation chamber 101 also tends to be consistent, thereby avoiding interference introduced by manual sample mixing and sample addition steps and improving the accuracy of the testing result.

The sample collected by the collection structure 1 may be an excrement, a secretion, or the like. The collection structure 1 may be an integrally formed structure or a structure formed from separate components. In order to facilitate disassembly and assembly, the collection structure 1 is designed as a structure having separate components.

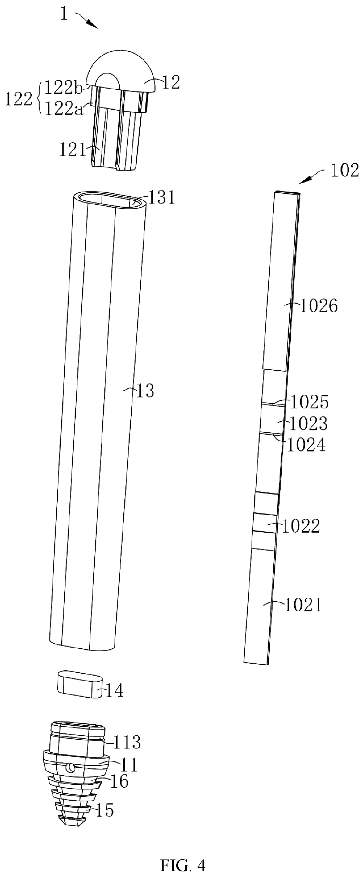

Referring to FIGS. 2 and 4, in some embodiments, the collection structure 1 includes a collection body 11, a cover body 12, and an accommodation body 13. The exterior of the collection body 11 is configured to collect a sample. The collection body 11 is provided with the liquid inlet 103. The accommodation body 13 has a hollow portion 131, the hollow portion 131 extending through the accommodation body 13 in a length direction of the accommodation body 13. The collection body 11 is detachably connected to one end of the hollow portion 131, and the cover body 12 is detachably connected to the other end of the hollow portion 131, such that the collection body 11, the cover body 12, and the accommodation body 13 jointly define the accommodation chamber 101.

Referring to FIG. 4, the accommodation body 13 can have a columnar shape, a box shape, a spherical shape, an ellipsoidal shape, a flat shape, or the like. Referring to FIG. 1, the accommodation body 13 can be entirely made of a transparent material or partially made of a transparent material, provided that the transparent material can serve as a viewing window 133 for observing the testing member 102 and does not interfere with reading of a testing result of the testing member 102.

Referring to FIG. 2, the cover body 12 and the accommodation body 13 can be rotatably connected through a threaded structure or can be engaged with each other through an engagement structure. Exemplarily, referring to FIG. 4, the cover body 12 includes a first engagement portion 122. The first engagement portion 122 includes a first neck portion 122a and a first shoulder portion 122b connected to each other. In a radial direction of the cover body 12, the first shoulder portion 122b is located outside the first neck portion 122a. The first neck portion 122a extends towards the interior of the hollow portion 131, and the first shoulder portion 122b extends towards the outside of the cover body 12, so as to increase respective connection areas of the first neck portion 122a and the first shoulder portion 122b with the accommodation body 13 and improve connection stability. When the cover body 12 is connected to the accommodation body 13, an outer peripheral surface of the first neck portion 122a of the cover body 12 abuts against a chamber wall of the accommodation body 13, and a bottom surface of the first shoulder portion 122b abuts against a top surface of the accommodation body 13.

Further, the cover body 12 is provided with an air hole extending therethrough for enabling the testing member 102 to perform chromatography. The air hole can also be provided in the accommodation body 13 of the collection structure 1.

Referring to FIG. 4, in some embodiments, the cover body 12 is further provided with a retaining groove 121. After the cover body 12 is connected to the accommodation body 13, the retaining groove 121 is in communication with the accommodation chamber 101, that is, the retaining groove 121 is located in the accommodation body 13. The retaining groove 121 is configured to fix the testing member 102. The width of the retaining groove 121 is consistent with the width of the testing member 102, such that the testing member 102 can be fixed more firmly and displacement of the testing member 102 can be prevented. The retaining groove 121 extends towards the interior of the hollow portion 131 so as to increase a fixing length of the testing member 102, thereby increasing a contact area with the testing member 102 and improving fixing stability. One or more retaining grooves 121 can be provided for fixing one or more testing members 102.

The collection body 11 has multiple functions. On one hand, the collection body 11 collects the sample. On another hand, the collection body 11 improves the mixing effect of the sample and the diluent. On a further hand, the collection body 11 serves as an inlet through which the liquid mixture enters the accommodation chamber 101 for testing. The exterior of the collection body 11 is inserted into a sample to be tested and then withdrawn, such that the sample can be collected on an outer surface of the collection body 11. The above operation can be repeated multiple times, such that a sufficient amount of the sample adheres to the collection body 11 for dilution.

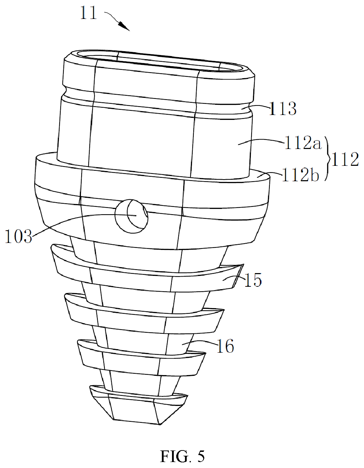

Referring to FIG. 4, the collection body 11 and the accommodation body 13 can also be rotatably connected through a helical structure or can be engaged with each other through an engagement structure. Referring to FIG. 5, exemplarily, referring to FIG. 5, the collection body 11 includes a second engagement portion 112. The second engagement portion 112 includes a second neck portion 112a and a second shoulder portion 112b. In a radial direction of the collection body 11, the second shoulder portion 112b is located outside the second neck portion 112a. The second shoulder portion 112b extends towards the interior of the hollow portion 131, and the second shoulder portion 112b extends towards the outside of the collection body 11, so as to increase an engagement area with the accommodation body 13. When the collection body 11 is engaged with the accommodation body 13, an outer peripheral surface of the second neck portion 112a abuts against the chamber wall of the accommodation body 13, and a top surface of the second shoulder portion 112b abuts against a bottom surface of the accommodation body 13.

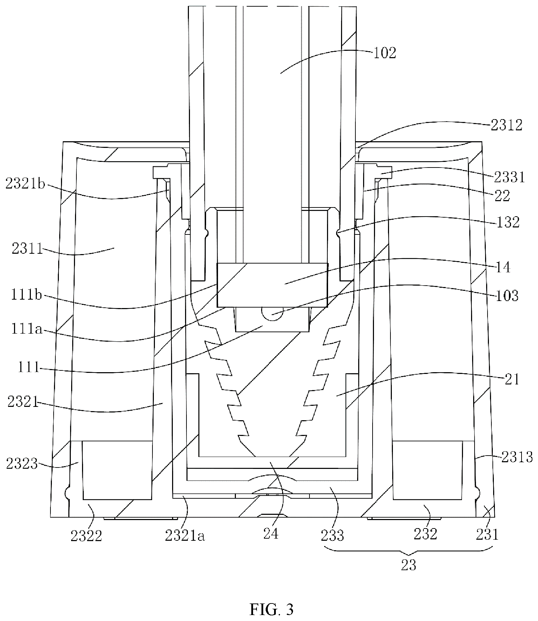

In order to further increase the connection stability between the collection body 11 and the accommodation body 13, referring to FIG. 3, the chamber wall of the accommodation body 13 is provided with a first protrusion 132 in a radial direction of the accommodation body 13, and referring to FIG. 5, the outer peripheral surface of the second neck portion 112a of the collection body 11 is provided with a first groove 113. Continuing to refer to FIG. 3, when the second neck portion 112a extends into the hollow portion 131 of the accommodation body 13, the first protrusion 132 is engaged with the first groove 113, thereby fixing the collection body 11 in an axial direction and preventing the collection body 11 from axially slipping out of the accommodation body 13.

Referring to FIG. 5, in a direction from a middle portion of the collection body 11 towards a bottom end thereof, a contour dimension of the collection body 11 gradually decreases to form a tapered shape, thereby reducing resistance when the collection body 11 is inserted into the liquid storage chamber 21 and facilitating insertion of the collection body 11 into the liquid storage structure 2. In addition, a tail end of the tapered collection body 11 is relatively narrow and is not easily blocked by solid particles or viscous substances, thereby ensuring that the liquid mixture smoothly enters the accommodation chamber 101 through the liquid inlet 103.

Referring to FIG. 5, an outer surface of the collection body 11 can be provided with a threaded structure or a toothed structure 15 to increase a contact area with the sample and improve an adhesion effect on the sample. Taking the toothed structure 15 as an example, on the exterior of the collection body 11, in a direction from the top end thereof towards the bottom end thereof, a plurality of toothed structures 15 are spaced apart and arranged in an array, and each toothed structure 15 is arranged around an outer periphery of the collection body 11. An annular groove 16 is formed between two vertically adjacent toothed structures 15, which, on one hand, can increase a contact area between the liquid and an inner wall of the annular groove 16, and increase contact and friction between the liquid and the collection body 11, so as to better mix the sample and the diluent. In addition, the annular groove 16 can change a flowing direction and a flowing speed of the liquid and increase turbulent movement of the liquid, thereby improving mixing efficiency, such that the diluent can flow back and forth in the annular groove 16 and continuously perform flushing and exchange, thereby improving a dilution effect of the sample.

Referring to FIG. 5, the liquid inlet 103 of the collection body 11 is disposed above the toothed structure 15 and is spaced apart from the toothed structure 15 by a certain distance, so as to avoid being blocked by the sample. The liquid inlet 103 is disposed in two opposite broad surfaces of the tapered collection body 11, which can ensure that the diluent can fully cover and mix with the sample on the outer surface of the collection body 11, thereby effectively flushing and mixing the sample, reducing sample residue, and improving the uniform mixing effect, and can facilitate smooth injection of the liquid mixture into the accommodation chamber 101.

Referring to FIG. 2, in some embodiments, the collection body 11 is recessed inward towards the bottom end thereof to form a recess 111 in communication with both the accommodation chamber 101 and the liquid inlet 103. Provision of the recess 111 can shorten a flowing distance of the liquid mixture, such that the liquid mixture can enter the accommodation chamber 101 from the collection body 11 more quickly, thereby improving testing efficiency.

Referring to FIG. 2, in some embodiments, the collection structure 1 further includes a filter body 14. The filter body 14 abuts against the chamber wall of the accommodation chamber 101. The filter body 14 is at least partially in contact with the liquid inlet 103 and the testing member 102. The filter body 14, on one hand, can buffer the liquid mixture, thereby preventing the liquid mixture from splashing, and enabling the liquid mixture to uniformly wet the testing member 102, and on the other hand, can filter insoluble solids or impurities in the sample, thereby making the concentration and composition of the liquid mixture more reliable and avoiding interference with testing by the testing member 102.

The filter body 14 can be made of a hydrophobic material having micropores that does not absorb the liquid mixture, such as ultra-high-molecular-weight polyethylene, polytetrafluoroethylene, polyolefin, or other materials. A pore diameter of the micropores in the filter body 14 is in a range of 20 µm to 80 µm. For example, the pore diameter may be any point value within the above range, such as 20 µm, 30 µm, 40 µm, 50 µm, 60 µm, 70 µm, 80 µm, etc.

Referring to FIG. 2, in some embodiments, the filter body 14 is positioned in the recess 111 of the collection body 11. The filter body 14 abuts against a wall of the recess 111. By positioning the filter body 14 in the collection body 11, the liquid mixture flowing into the recess 111 through the liquid inlet 103 is timely filtered by the filter body 14, thereby improving the uniformity and consistency of the liquid mixture.

Referring to FIG. 3, in some embodiments, a recess body of the recess 111 includes a stepped portion 111a and an abutting portion 111b. The abutting portion 111b is disposed at a top end of the stepped portion 111a. The filter body 14 simultaneously abuts against an upper surface of the stepped portion 111a and an inner peripheral surface of the abutting portion 111b. The filter body 14 is located above the liquid inlet 103. The testing member 102 abuts against an upper surface of the filter body 14. The stepped portion 111a is provided such that a certain distance is maintained between the filter body 14 and the liquid inlet 103, thereby avoiding interference with entry of the liquid mixture into the recess 111 and enabling the liquid mixture to enter the recess 111 more smoothly.

The testing member 102 in the embodiments of the present application can adopt a colloidal-gold lateral-flow chromatographic structure, a fluorescence lateral-flow chromatographic structure, a quantum-dot lateral-flow chromatographic mechanism, an enzymatic-reaction vertical-flow chromatographic mechanism, or a microchannel siphon-reaction structure. The above testing member 102 can utilize an antigen-antibody immunological reaction and a chromatographic reaction, thereby achieving rapid and accurate color development for testing a target substance. The testing member 102 can test various types of analytes and can employ a non-competitive assay method or a competitive assay method.

Referring to FIG. 4, the testing member 102 can test various types of analytes and can adopt a non-competitive assay mode or a competitive assay mode. The testing member 102 includes a sample pad 1021 made of a water-absorbing material for absorbing and receiving a sample, a protein conjugate pad 1022 coated with a label, a nitrocellulose membrane 1023 including a testing line 1024 coated with an antibody and a quality control line 1025 coated with an antibody, and a water-absorbing pad 1026 having a water-absorbing function. When the testing member 102 is disposed in the collection structure 1, the absorbing paper is inserted into the retaining groove 121 of the cover body 12, the sample pad 1021 comes into contact with the filter body 14 in the collection body 11, and the testing line 1024 and the quality control line 1025 of the strip of the testing member 102 correspond to the viewing window 133 of the accommodation body 13, so as to read a testing result.

The testing principle of the testing member 102 is to use immunochromatographic technology and use specific binding between immune molecules to test whether an analyte is present in the sample. The liquid mixture undergoes chromatography under a capillary force. When the liquid mixture passes through the protein conjugate pad 1022, the analyte in the liquid mixture undergoes a specific immune reaction with an immune complex in the protein conjugate pad 1022 to produce an antigen-antibody complex. Under continuous chromatography, the antigen-antibody complex is captured by the testing line 1024 and the quality control line 1025 coated on the nitrocellulose membrane 1023 to form testing bands. The appearance of the testing line 1024 indicates that the analyte is present in the sample being tested.

The analyte in the liquid mixture may be hemoglobin or transferrin, by which it may be determined that the subject has gastrointestinal bleeding. Alternatively, the analyte in the liquid mixture may be Helicobacter pylori, by which it may be determined that the subject has a Helicobacter pylori infection. Further alternatively, the analyte in the liquid mixture may be rotavirus or adenovirus, by which it may be determined that the subject has a rotavirus or adenovirus infection.

The testing member 102 may include multiple materials for transferring the liquid sample. Exemplarily, a material in the testing member 102 should partially stack on and cover an adjacent material. For example, the protein conjugate pad 1022 should cover the nitrocellulose membrane 1023. Various components of the strip of the testing member 102 may employ one or more materials. Each material may be adhered to a rigid surface or a support so as to improve the overall strength of the testing member 102.

The testing member 102 may be present in the accommodation chamber 101 in various forms and configurations. For example, the testing member 102 may be suspended at the center of the accommodation chamber 101 or closely attached to one side of the chamber wall of the accommodation chamber 101, or a rigid support may be disposed in the middle of the accommodation chamber 101 to fix the testing member 102. The testing member 102 may be configured to test a single testing item or multiple testing items. The number of strips of the testing members 102 in the accommodation chamber 101 may be one or more.

Referring to FIGS. 1 and 2, the liquid storage structure 2 serves as a storage place for the diluent, and the access opening 22 thereof is sealed by a sealing sheet. The sealing sheet can be an aluminum foil, plastic, or the like. The collection body 11 of the collection structure 1 pierces the sealing sheet and is inserted into the liquid storage chamber 21.

Referring to FIG. 2, after the collection structure 1 enters the liquid storage chamber 21, the accommodation body 13 of the collection structure 1 needs to closely fit against the chamber wall of the liquid storage chamber 21, so as to prevent liquid leakage, while generating an impact force for mixing the sample with the diluent and causing the liquid mixture to be squeezed to flow into the liquid inlet 103. In order to achieve the interference fit between the accommodation body 13 and the chamber wall of the accommodation chamber 101, the following manners may be employed, including but not limited to: the hard materials of the two may press against each other to form a seal, or a sealing ring may be fixed on the chamber wall of the liquid storage chamber 21, such that the accommodation body 13 is sealed against the chamber wall.

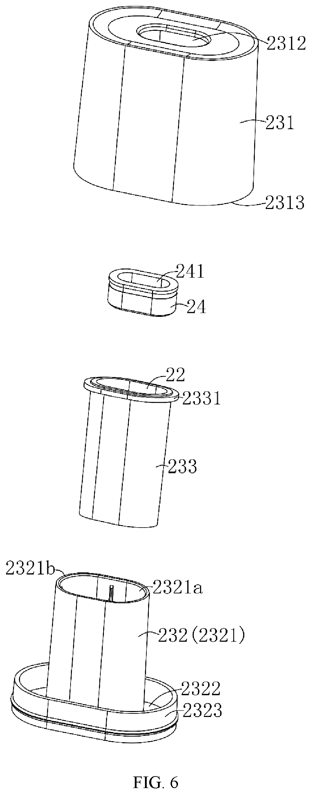

Referring to FIGS. 3 and 6, in some embodiments, the liquid storage structure 2 includes a base 23 and a pressurizing member 24. The base 23 has the liquid storage chamber 21 and the access opening 22. The pressurizing member 24 is disposed in the liquid storage chamber 21 and can movably abut against the chamber wall of the liquid storage chamber 21. The pressurizing member 24 is provided with a through hole 241 extending therethrough and in communication with the liquid storage chamber 21. The collection structure 1 is configured to enter the liquid storage chamber 21 and push the pressurizing member 24 to move relative to the chamber wall, such that the pressurizing member 24 squeezes the diluent to cause the diluent to flush the sample to form the liquid mixture, and further causes the liquid mixture to rush out through the through hole 241 and enter the accommodation chamber 101 through the liquid inlet 103. By disposing the pressurizing member 24 in the liquid storage chamber 21, the contact area with the diluent is further increased on the basis of the collection structure 1, the pressurizing effect on the diluent is improved, and the diluent can be sprayed so as to be better mixed with the sample.

Referring to FIG. 6, specifically, the pressurizing member 24 has an annular structure with an L-shaped axial cross-section. The pressurizing member 24 includes a first portion and a second portion connected at an angle. The first portion is disposed along an axial direction of the liquid storage chamber 21. The second portion is disposed along a radial direction of the liquid storage chamber 21. The second portion is provided with a through hole 241 extending therethrough in a thickness direction thereof. One or more through holes 241 may be provided. When the pressurizing member 24 abuts against the chamber wall of the liquid storage chamber 21, an outer peripheral surface of the first portion abuts against the chamber wall of the liquid storage chamber 21. Accordingly, compared with a flat structure, the pressurizing member 24 has a concave structure, which can increase the strength and stability of the pressurizing member 24, enable the pressurizing member 24 to withstand greater pressure, and reduce deformation and distortion. The provision of the first portion increases a contact area between the pressurizing member 24 and the chamber wall of the liquid storage chamber 21, provides a better abutting effect between the pressurizing member 24 and the liquid storage chamber 21, enables a stable squeezing force to be applied to the liquid mixture, and provides a better pressurizing effect on the liquid mixture.

It should be noted that, before the collection structure 1 is inserted into the liquid storage chamber 21, the pressurizing member 24 is disposed at an intermediate position of the liquid storage chamber 21 in the axial direction, and the second portion is suspended in the liquid storage chamber 21. After the collection structure 1 is inserted into the liquid storage chamber 21, the pressurizing member 24 gradually moves towards the bottom end of the liquid storage chamber 21 until the second portion abuts against the bottom end of the liquid storage chamber 21.

The base 23 is configured, on one hand, to store the diluent and, and on the other hand, to provide stable support when the collection structure 1 is inserted into the liquid storage chamber 21. The base 23 may be a one-piece structure or a multi-piece structure. Referring to FIGS. 3 and 6, in some embodiments, the base 23 includes an upper base cover 231, a lower base cover 232, and a diluent tube 233.

Referring to FIG. 3, the diluent tube 233 has the liquid storage chamber 21 and the access opening 22. The diluent tube 233 serves as a place for directly storing the diluent and directly interference-fits with the collection structure 1. The pressurizing member 24 is disposed in the diluent tube 233 and abuts against an inner wall of the diluent tube 233.

Referring to FIG. 3, the lower base cover 232 includes a main body portion 2321 and an extension portion 2322. The extension portion 2322 is connected to an outer periphery of the main body portion 2321 and extends in a direction away from the main body portion 2321. The main body portion 2321 has a first placement chamber 2321a and a first opening 2321b in communication with the first placement chamber 2321a.

Referring to FIG. 3, the upper base cover 231 has a second placement chamber 2311, and a second opening 2312 and a third opening 2313 that are in communication with the second placement chamber 2311. The second opening 2312 and the third opening 2313 are oppositely disposed in the axial direction of the base 23.

Referring to FIG. 3, the diluent tube 233 extends through the first opening 2321b and is positioned in the first placement chamber 2321a. An outer wall of the diluent tube 233 abuts against the chamber wall of the first placement chamber 2321a. The lower base cover 232 extends through the third opening 2313 and is positioned in the second placement chamber 2311. The outer periphery of the extension portion 2322 abuts against the chamber wall of the second placement chamber 2311. The access opening 22 is at least partially exposed through the first opening 2321b and the second opening 2312. In other words, during assembly of the base 23, the diluent tube 233 is inserted into the first placement chamber 2321a of the lower base cover 232, and then the upper base cover 231 is engaged with the lower base cover 232.

Referring to FIG. 3, further, a portion of the diluent tube 233 at the access opening 22 protrudes outward from the outer periphery of the diluent tube 233 to form a lapping portion 2331. When the diluent tube 233 is inserted into the first placement chamber 2321a of the lower base cover 232, the lapping portion 2331 is lapped on the top end of the lower base cover 232.

Referring to FIG. 3, the extension portion 2322 of the lower base cover 232 can enlarge a support area of the base 23 in the radial direction, thereby improving the support stability of the liquid storage structure 2. The extension portion 2322 is an L-shaped structure. The extension portion 2322 extends along the radial direction of the lower base cover 232, and then bents and extends upwardly to form a bent portion 2323. The bent portion 2323 can increase the connection area with the upper base cover 231 and improve the engagement effect between the upper base cover 231 and the lower base cover 232.

When the upper base cover 231 and the lower base cover 232 are fitted together, a portion of the upper base cover 231 adjacent to the second opening 2312 abuts against the lapping portion 2331 of the diluent tube 233, thereby pressing the diluent tube 233 in the axial direction. A portion of the upper base cover 231 adjacent to the third opening 2313 abuts against the bent portion 2323 of the lower base cover 232, thereby fixing the lower base cover 232 and the diluent tube 233 in the radial direction. Accordingly, the upper base cover 231 can sufficiently fix the diluent tube 233 in both the axial direction and the radial direction, ensure the overall stability of the liquid storage structure 2, and prevent the diluent tube 233 from becoming loose or detached.

Referring to FIG. 3, the upper base cover 231 is provided with the second placement chamber 2311, and the second placement chamber 2311 cooperates with the extension portion 2322 and the bent portion 2323 of the lower base cover 232, such that the upper base cover 231 has a hollow structure. On one hand, the hollow structure can accommodate the lower base cover 232 and the diluent tube 233. On the other hand, the hollow structure can reduce the weight of the upper base cover 231, thereby reducing the overall weight of the base 23 and facilitating carrying. Further, the hollow structure can lower the center of gravity of the base 23 and ensure placement stability during testing.

Referring to FIGS. 2 and 3, an assembly method for the sample collection and testing device 100 provided in the embodiments of the present application will be specifically described below. The diluent tube 233 in which the diluent has been sealed is inserted into the first placement chamber 2321a of the lower base cover 232 with the access opening 22 oriented upwards, and then the upper base cover 231 and the lower base cover 232 are pressed into engagement, thereby completing assembly of the liquid storage structure 2. The filter body 14 is pushed into the recess 111 in the collection body 11, such that the filter body 14 abuts against and is pressed against the stepped portion 111a and the abutting portion 111b. Next, the collection body 11 is engaged with the bottom end of the hollow portion 131 of the accommodation body 13, the testing member 102 is then retained in the retaining groove 121 of the cover body 12, and the cover body 12 is engaged with the top end of the hollow portion 131 of the accommodation body 13, such that the testing member 102 is in direct contact with the filter body 14, thereby completing assembly of the collection structure 1.

In summary, the sample collection and testing device 100 provided in the embodiments of the present application has at least the following technical effects. First, automatic mixing is achieved, without requiring additional manual shaking for sample mixing or a manual sample addition operation, the testing steps are simple, the number of times and duration for which the subject contacts the sample are reduced, the testing efficiency and testing accuracy are improved, and the testing result is prevented from being affected by excessive testing steps. Second, the sample collection and testing device is reliable, and the diluent and the testing member are independently located in different spaces, thereby preventing the testing member from being wetted by the diluent and becoming ineffective. Third, the sample collection and testing device has a compact and refined structure, thereby improving space utilization rate. After use, the collection structure and the liquid storage structure are tightly connected to form an integral whole, such that the liquid mixture is enclosed in the device, thereby reducing the secondary contamination and facilitating the centralized collection and disposal. Fourth, as a household self-testing product, compared with testing in a large medical institution, the product has lower requirements in term of time and space, can be used for testing whenever needed, enable timely detection of issues, and can prompt the subject to seek medical attention in a timely manner. Fifth, the testing result is not affected by the operating habits of the subject, the learning cost is low, and the device is suitable for infants or elderly persons, thereby improving the user experience.

In a second aspect, the embodiments of the present application further provide a kit. The kit includes a sample collection and testing device 100. The sample collection and testing device 100 may be the sample collection and testing device 100 described in the first aspect above. One, two, or another number of sample collection and testing devices 100 may be provided in the kit. The kit can be used to test whether a subject is infected with Helicobacter pylori or whether fecal occult blood is present.

In a third aspect, the embodiments of the present application further provide a testing method. The testing method tests a sample by using a sample collection and testing device 100. The sample collection and testing device 100 is the sample collection and testing device 100 according to the first aspect. The testing method includes the following steps: adhering a sample to the collection structure 1; inserting the collection structure 1 having the sample adhered thereto into the liquid storage chamber 21 through the access opening 22, squeezing the diluent in the liquid storage chamber 21 to cause the diluent to flush the sample to form a liquid mixture, and pressing the liquid mixture into the accommodation chamber 101 to come into contact with the testing member 102.

The testing member in the accommodation chamber of the collection structure is observed, and the testing result is read.

The solution of the present application will be specifically described below by way of specific examples and comparative examples.

### Example 1

Example 1 provided a kit for testing a Helicobacter pylori antigen. The kit included a sample collection and testing device. The sample collection and testing device included a collection structure and a liquid storage structure. The accommodation chamber of the collection structure was provided with a Helicobacter pylori test strip therein. A testing line on a nitrocellulose membrane of the Helicobacter pylori test strip was coated with a Helicobacter pylori-specific antibody, a quality control line on the nitrocellulose membrane was coated with a goat anti-mouse antibody, and a protein conjugate pad of the Helicobacter pylori test strip was coated with a Helicobacter pylori-specific antibody labelled with colloidal gold.

### Example 2

Example 2 provided a kit for combined testing of fecal occult blood/transferrin. The kit included a sample collection and testing device. The sample collection and testing device included a collection structure and a liquid storage structure. The accommodation chamber of the collection structure was provided with two test strips therein, one being a fecal occult blood test strip and the other being a transferrin test strip. A testing line on a nitrocellulose membrane of the fecal occult blood test strip was coated with an anti-haemoglobin-specific antibody, a quality control line on the nitrocellulose membrane was coated with a goat anti-mouse antibody, and a protein conjugate pad of the fecal occult blood test strip was coated with an anti-haemoglobin-specific antibody labelled with colloidal gold. A testing line on a nitrocellulose membrane of the transferrin test strip was coated with an anti-transferrin-specific antibody, a quality control line on the nitrocellulose membrane was coated with a goat anti-mouse antibody, and a protein conjugate pad of the transferrin test strip was coated with an anti-transferrin-specific antibody labelled with colloidal gold.

### Comparative Example 1

Comparative Example 1 provided a kit for testing a Helicobacter pylori antigen. The kit included a sampling stick, a sample dilution tube containing a diluent, and a Helicobacter pylori antigen testing plate provided with a sample addition well.

### Comparative Example 2

Comparative Example 2 provided a kit for combined testing of fecal occult blood/transferrin. The kit included a sampling stick, a sample dilution tube containing a diluent, and a fecal occult blood/transferrin testing plate provided with a sample addition well.

### Testing and Result Analysis

The collection structures of the sample collection and testing devices in Example 1 and Example 2 were each inserted into a fecal sample to be tested. Each collection structure was inserted into the liquid storage chamber and pressed down until the pressurizing member was in contact with the bottom end of the liquid storage chamber, and was then allowed to stand until the result was read.

The sampling sticks in the kits of Comparative Example 1 and Comparative Example 2 were each inserted into a fecal sample to be tested. The sampling sticks were then placed into the sample dilution tubes containing the diluent, and the sample dilution tubes were tightened, shaken to mix uniformly, and allowed to stand for a period of time. After standing was completed, the sampling sticks were removed, then the sample dilution tubes were tightened, and two to three drops of the liquid mixture were vertically added dropwise to the liquid addition wells of the respective testing plates. The testing plates were allowed to stand and wait for the result to be read.

**Table 1**

| Category | Testing operation steps | Operation time | Total testing time | Safety |
|---|---|---|---|---|
| Exmaple 1 | Sampling and pressing | 5s to 10 s | 10-15 min | The diluent and the testing member were separately provided, with a good product stability. |
| Exmaple 2 | Sampling and pressing | 5 s to 10 s | 10-15 min | The diluent and the testing member were separately provided, with a good product stability. |
| Comparative Example 1 | Sampling, shaking to mix uniformly, standing, and dropwise addition | 3 min to 5 min | 18-30 min | The liquid mixture was prone to flowing out during dropwise addition, causing contamination. |
| Comparative Example 2 | Sampling, shaking to mix uniformly, standing, and dropwise addition | 3 min to 5 min | 18-30 min | The liquid mixture was prone to flowing out during dropwise addition, causing contamination. |

### Result Analysis

It can be intuitively seen from the results in Table 1 that, compared with Comparative Example 1 and Comparative Example 2, Example 1 and Example 2 of the present application have significantly fewer testing operation steps, the operation time is shortened from several minutes to several seconds, and the total testing time is greatly shortened. Compared with conventional household self-testing products, the sample collection and testing device provided in the embodiments of the present application has simple operation steps, high testing efficiency, and better safety.

In the present application, unless otherwise expressly specified and defined, where descriptions such as a first feature being "on" or "under" a second feature appear, the descriptions may mean that the first feature and the second feature are in direct contact, or that the first feature and the second feature are in indirect contact through an intermediate medium. Moreover, the first feature being "above", "over", or "on top of" the second feature may mean that the first feature is directly above or obliquely above the second feature, or may merely mean that a horizontal height of the first feature is greater than that of the second feature. The first feature being "below", "under", or "beneath" the second feature may mean that the first feature is directly below or obliquely below the second feature, or may merely mean that a horizontal height of the first feature is less than that of the second feature.

It should be noted that, when an element is referred to as being "fixed to" or "disposed on" another element, the element may be directly on the other element, or an intermediate element may also be present. When an element is referred to as being "connected to" another element, the element may be directly connected to the other element, or an intermediate element may also be present therebetween. Where present, the terms "vertical", "horizontal", "upper", "lower", "left", "right", and similar expressions used in the present application are merely for illustrative purposes and do not indicate the only implementation.

The technical features of the above-mentioned embodiments can be combined arbitrarily. In order to make the description concise, not all possible combinations of the technical features of the above-mentioned embodiments are described. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be noted that a person of ordinary skill in the art may make various modifications and improvements without departing from the concept of the present application, and all such modifications and improvements fall within the scope of protection of the present application. Therefore, the scope of patent protection of the present application shall be defined by the appended claims.

## Claims

1. A sample collection and testing device, **characterized by** comprising:
a collection structure having an accommodation chamber, wherein the accommodation chamber is configured to accommodate a testing member, an end of the collection structure is configured to collect a sample, and the collection structure is provided with a liquid inlet in communication with the accommodation chamber; and
a liquid storage structure having a liquid storage chamber and an access opening in communication with the liquid storage chamber, wherein the liquid storage chamber is configured to store a diluent, and the collection structure is configured to enter the liquid storage chamber through the access opening, be in interference fit with a chamber wall of the liquid storage chamber, and move along the chamber wall.

2. The sample collection and testing device according to claim 1, **characterized in that** the liquid storage structure comprises a base and a pressurizing member, the base has the liquid storage chamber and the access opening, the pressurizing member is disposed in the liquid storage chamber and is movably abutting against the chamber wall, the pressurizing member is provided therein with a through hole extending through the pressurizing member and in communication with the liquid storage chamber, and the collection structure is configured to enter the liquid storage chamber and push the pressurizing member to move relative to the chamber wall.

3. The sample collection and testing device according to claim 2, **characterized in that** the base comprises an upper base cover, a lower base cover, and a diluent tube;
the lower base cover comprises a main body portion and an extension portion, the extension portion is connected to an outer periphery of the main body portion and extends in a direction away from the main body portion, the main body portion has a first placement chamber and a first opening in communication with the first placement chamber, the upper base cover has a second placement chamber and a second opening and a third opening that are in communication with the second placement chamber, the second opening and the third opening are oppositely disposed in an axial direction of the upper base cover, the diluent tube has the liquid storage chamber and the access opening, and the pressurizing member is disposed in the diluent tube and abuts against an inner wall of the diluent tube; and
the diluent tube extends through the first opening and is positioned in the first placement chamber, an outer wall of the diluent tube abuts against a chamber wall of the first placement chamber, the lower base cover extends through the third opening and is positioned in the second placement chamber, an outer periphery of the extension portion abuts against a chamber wall of the second placement chamber, and the access opening is at least partially exposed through the first opening and the second opening.

4. The sample collection and testing device according to claim 1, **characterized in that** the collection structure comprises a collection body, a cover body, and an accommodation body, an end of the collection body is configured to collect the sample, the collection body is provided with the liquid inlet, and the accommodation body has a hollow portion extending therethrough in a length direction thereof; and
the collection body is detachably connected to one end of the hollow portion, and the cover body is detachably connected to another end of the hollow portion, such that the collection body, the cover body, and the accommodation body jointly define the accommodation chamber.

5. The sample collection and testing device according to claim 4, **characterized in that** the collection structure further comprises a filter body, the filter body abuts against the chamber wall of the accommodation body, and the filter body is at least partially in contact with the liquid inlet and the testing member.

6. The sample collection and testing device according to claim 5, **characterized in that** the collection body is recessed inward in a direction towards a bottom end thereof to form a recess, the recess is in communication with both the accommodation chamber and the liquid inlet, the filter body is located in the recess, and the filter body abuts against a wall of the recess.

7. The sample collection and testing device according to claim 6, **characterized in that** a recess body of the recess comprises a stepped portion and an abutting portion, the abutting portion is disposed at a top end of the stepped portion, the filter body abuts against both an upper surface of the stepped portion and an inner peripheral surface of the abutting portion, the filter body is located above the liquid inlet, and the testing member abuts against an upper surface of the filter body.

8. The sample collection and testing device according to claim 4, **characterized in that** the cover body is provided with a retaining groove, and after the cover body is connected to the accommodation body, the retaining groove is in communication with the accommodation chamber, and the retaining groove is configured to fix the testing member.

9. A kit, **characterized by** comprising the sample collection and testing device according to any one of claims 1 to 8.

10. A testing method, **characterized by** using the sample collection and testing device according to any one of claims 1 to 8, wherein the testing method comprising the following steps:
adhering a sample to the collection structure;
inserting the collection structure having the sample adhered thereto into the liquid storage chamber through the access opening, squeezing the diluent in the liquid storage chamber to cause the diluent to flush the sample to form a liquid mixture, and pressing the liquid mixture into the accommodation chamber to be in contact with the testing member; and
observing the testing member in the accommodation chamber of the collection structure and reading a testing result.
